# EUROPEAN PATENT APPLICATION

(11) **EP 4 488 298 A1**
(43) Date of publication of application: **08.01.2025**
(21) Application number: 23759816.4
(22) Date of filing: 15.02.2023
(51) Int. Cl.: C08B 37/16, B01J 20/22, B01J 20/28, B01J 20/30, C07C 63/307, C07D 233/58

(54) **METAL-ORGANIC FRAMEWORK**

(30) Priority: 28.02.2022 JP 2022029433; 28.02.2022 JP 2022029486; 26.05.2022 JP 2022086235
(71) Applicant: Toyo Seikan Group Holdings, Ltd., Shinagawa-ku Tokyo 141-8627 (JP)
(72) Inventor: TSURUTA, Kazuhiro, Yokohama-shi, Kanagawa 240-0062 (JP); MIYAI, Tomohiro, Yokohama-shi, Kanagawa 240-0062 (JP)
(74) Representative: Marks & Clerk LLP
(86) International application number: PCT/JP2023/005204
(87) International publication number: WO 2023/162815

(57) **Abstract**

The purpose of the present invention is to provide a metal-organic framework having excellent gas adsorption ability. The present invention provides a metal-organic framework which comprises a metal ion and an organic ligand coordinated to the metal ion, and has a cyclic structure or a cage-like structure, wherein the metal ion is bound in an amount of 0.74 moles or less per 1 mole of the organic ligand.

## Description

### Technical Field

The present invention relates to a metal-organic framework.

### Background Art

Metal-organic frameworks (MOFs) are materials formed by coordination bonds between metals and organic ligands. As such metal-organic frameworks, porous materials having a cyclic structure capable of adsorbing guest molecules are known. Such metal-organic frameworks have advantages such as easier control of the pore size than porous materials such as zeolite and activated carbon, and are expected to be practically used in various applications such as gas adsorbents.

In connection with metal-organic frameworks having a cyclic structure, Non-Patent Literature 1 (Yaghi, O. M., Angew. Chem. Int. Ed. Engl. 2010, 49, 8630-8634) discloses a method of producing a metal-organic framework using γ-cyclodextrin. Non-Patent Literature 1 states that a metal-organic framework was obtained by mixing 1.0 equivalent of γ-cyclodextrin with 8 equivalents of KOH in an aqueous solution, and then subjecting the aqueous solution to "vapor diffusion" of methanol for 2 to 7 days.

Further, Patent Literature 1 (Japanese Patent Application Publication No. 2021-54490) proposes a gas-absorbing film containing a thermoplastic resin and a gas absorbent, wherein the gas absorbent contains a gas physical absorbent and/or a gas chemical absorbent; a BET specific surface area of the gas physical absorbent is 50 m²/g or more and 4000 m²/g or less; and a content of the gas absorbent in a layer containing the gas absorbent is 0.5 mass% or more and 30 mass% or less, and states that a metal-organic framework can be used as one of the gas physical absorbents in this gas-absorbing film.

Patent Literature 1 states that using a specific metal-organic framework such as 2-methylimidazole zinc, it was possible to adsorb organic gases such as butane, carbon dioxide, aldehyde, and acetic acid.

Since the pore size and the shape of the structure of the metal-organic framework differ depending on the type and combination of the constituent metal ions and organic ligands, or the coordination ratio in the synthesis thereof, and the substances that can be adsorbed also differ, a new adsorbent using a metal-organic framework capable of adsorbing substances other than the above organic gases is expected.

### Citation List

### Patent Literatures

Patent Literature 1: Japanese Patent Application Publication No. 2021-54490

### Non Patent Literatures

Non-Patent Literature 1: Yaghi, O.M., Angew. Chem. Int. Ed. Engl. 2010, 49, 8630-8634

### Summary of Invention

It would be advantageous if a substance having a superior adsorption capacity could be obtained as the metal-organic framework. Therefore, an object of the present invention is to provide a metal-organic framework having excellent adsorption capacity.

As a result of intensive studies, the present inventors have found that the above object can be achieved by the following means.
[1] A metal-organic framework comprising a metal and an organic ligand coordinated to the metal, wherein the metal-organic framework has a cyclic structure or a cage-like structure, and the metal is bonded in an amount of 7.40 mol or less relative to 1 mol of the organic ligand.
[2] The metal-organic framework according to [1], wherein a nitrogen gas adsorption amount is 220 cm³/g or more.
[3] The metal-organic framework according to [1] or [2], wherein a CO₂ gas adsorption amount is 10 cm³/g or more.
[4] The organometallic framework according to any one of [1] to [3], wherein the metal is an alkali metal.
[5] The organometallic framework according to [4], wherein the alkali metal is potassium.
[6] The organometallic framework according to any one of [1] to [5], wherein the organic ligand is cyclodextrin.
[7] The organometallic framework according to [6], wherein the cyclodextrin is γ-cyclodextrin.
[8] A gas adsorbent comprising the organometallic framework according to any one of [1] to [7].
[9] An adsorbent capable of adsorbing at least a monocyclic compound, wherein the adsorbent contains a metal-organic framework having a cyclic structure or a cage-like structure formed by coordinating at least one organic ligand to at least one metal ion, and the organic ligand is a cyclic organic compound.
[10] The adsorbent according to [9], wherein the metal ion is an alkali metal ion.
[11] The adsorbent according to [10], wherein the alkali metal is potassium.
[12] The adsorbent according to any one of [9] to [12], wherein the cyclic organic compound is a cyclodextrin-based compound.
[13] The adsorbent according to [12], wherein the cyclodextrin-based compound is α-cyclodextrin or γ-cyclodextrin.
[14] The adsorbent according to any one of [9] to [14], wherein the organic ligand of the metal-organic framework is cyclodextrin, and the metal ion is a potassium ion.
[15] The adsorbent according to any one of [9] to [14], wherein the metal-organic framework has a two-dimensional sheet-like layered structure.
[16] The adsorbent according to any one of[9] to [15], wherein the monocyclic compound is a cyclopentane derivative.
[17] The adsorbent according to any one of [9] to [16], wherein the metal ion is bonded in an amount of 7.40 mol or less relative to 1 mol of the organic ligand.
[18] A resin composition comprising the adsorbent according to any one of [9] to [17] in a resin.
[19] A method of producing a metal-organic framework having a cyclic structure, the method comprising: preparing an aqueous solution containing a metal ion and an organic ligand capable of coordinating to the metal ion as a first solution; and directly adding a second solution containing an organic solvent to the first solution to produce a metal-organic framework in which the organic ligand is coordinated to the metal ion.
[20] The production method according to [19], wherein the metal ion is bonded in an amount of 7.40 mol or less relative to 1 mol of the organic ligand.
[21] The production method according to [19] or [20], wherein the metal ion is an alkali metal ion.
[22] The production method according to [21], wherein the metal ion is a potassium ion.
[23] The production method according to any one of [19] to [22], wherein the organic ligand includes a cyclodextrin-based compound.
[24] The production method according to [23], wherein the cyclodextrin-based compound includes γ-cyclodextrin.
[25] The production method according to any one of [19] to [24], wherein the metal ion is a zinc ion, and the organic ligand is a compound having an imidazole backbone.
[26] The production method according to [25], wherein the compound having an imidazole backbone is 2-methylimidazole.
[27] The production method according to any one of [19] to [26], wherein the metal ion is an iron ion, and the organic ligand is a compound having a monocyclic or polycyclic backbone.
[28] The production method according to [27], wherein the compound having a monocyclic or polycyclic backbone is trimesic acid.
[29] The production method according to claim 19, wherein the organic ligand is used in an amount of 0.05 to 50 mol relative to 1 mol of the metal ion in the first solution.
[30] The production method according to any one of [19] to [29], wherein the second solution is a solution that changes the pH of the first solution.
[31] The production method according to [30], wherein the second solution is a solution that lowers the pH of the first solution, and the second solution is added in an amount so that the pH of the first solution decreases by 0.01 to 0.20.
[32] The production method according to any one of [19] to [31], wherein the second solution contains at least one solvent selected from the group consisting of an alcohol-based solvent, a ketone-based solvent, and an aprotic solvent.
[33] The production method according to any one of [19] to [32], wherein in the step of producing the metal-organic framework, the second solution is added to the first solution while applying shear to the first solution.
[34] The production method according to any one of [19] to [33], further comprising isolating the metal-organic framework after the step of producing the metal-organic framework.
[35] The production method according to [34], wherein the isolating step includes filtering a mixed solution of the first solution and the second solution.
[36] The production method according to [35], wherein the isolating step includes washing or drying the metal-organic framework after the filtering step.

According to the present invention, a metal-organic framework having excellent gas adsorption capacity is provided.

### Description of Embodiments

Hereinafter, embodiments of the present invention will be described.

### 1: Metal-Organic Framework

The metal-organic framework according to the present embodiment is a structure containing a metal ion and an organic ligand coordinated to the metal ion. The metal-organic framework has a cyclic structure or a cage-like structure. Further, the metal ion is bonded in an amount of 7.40 mol or less relative to 1 mol of the organic ligand. According to the knowledge of the present inventors, by having such a configuration, a metal-organic framework excellent in adsorption capacity is achieved.

The "cyclic structure or cage-like structure" specifically refers to a cyclic structure or a cage-like structure having a space capable of encapsulating a guest molecule. By having such a structure, the guest molecule can be adsorbed to the metal-organic framework.

The cyclic structure is preferably a structure formed by bonding a metal ion and an organic ligand.

The cage-like structure means a cubic crystal structure obtained by further advancing crystallization of a metal-organic framework having a cyclic structure formed by bonding a metal ion and an organic ligand.

The presence of a cyclic structure or a cage-like structure can impart a function of adsorbing a guest molecule to the metal-organic framework. Due to this function, the metal-organic framework can be used in applications where a function of adsorbing a substance is required (for example, a gas adsorbent).

The number of moles of the metal ion relative to 1 mol of the organic ligand may be 7.40 mol or less, preferably 3.50 to 7.40 mol, more preferably 5.00 to 7.35 mol, and further preferably 6.00 to 7.30 mol.

The metal ion may be any as long as it can form a coordination bond with the organic ligand. Examples of the metal ion include at least one metal ion selected from the group consisting of Li, Na, K, Rb, Be, Mg, Ca, Sr, Ba, Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Pb, As, Sb, and Bi. Among these, alkali metal ions, Zn ions, and Fe ions are preferred. As the alkali metal ion, potassium ion and sodium ion are more preferred. As the alkali metal ion, potassium ion is most preferred.

The organic ligand may be any as long as it forms a coordination bond with the metal ion. As the organic ligand, a compound having a functional group that forms a coordination bond with the metal ion can be used. Examples of such a functional group include a hydroxyl group, an imidazole group, a pyridyl group, a carboxyl group, a sulfonic acid group, and an amide group.

In one aspect, as the organic ligand, a compound having a structure capable of encapsulating a guest molecule can be used. Examples of such a compound include cyclodextrin-based compounds and crown ethers. Crown ethers are cyclic oligomers of ethylene glycol containing carbon, hydrogen, and oxygen, in which oxygen atoms are each bonded to two carbon atoms to form a crown-like ring. The organic ligand is preferably a cyclodextrin-based compound, and examples thereof include α-cyclodextrin, β-cyclodextrin, and γ-cyclodextrin. When γ-cyclodextrin is used, a metal-organic framework excellent in nitrogen adsorption properties and carbon dioxide (CO₂) gas adsorption properties can be achieved. Additionally, a metal-organic framework employing α-cyclodextrin as the organic ligand has a two-dimensional, sheet-like layered structure with adsorption properties for heptane, octane, and other non-monocyclic compounds, which a cage-like structure metal-organic framework utilizing γ-cyclodextrin as the organic ligand could not adsorb, as is clear from the results of the Examples described later.

In the present invention, as the metal-organic framework to be used, when the organic ligand is a cyclodextrin-based compound and the metal ion is a potassium ion, the metal-organic framework in which the cyclodextrin-based compound is bonded in the range of 0.05 to 0.8 mol relative to 1 mol of potassium ions is suitable for exhibiting excellent adsorption performance.

In other words, as is clear from the results of Examples described later, the adsorbent (Example 1) using γ-CD-MOF in which γ-cyclodextrin was mixed in an amount of 0.125 mol relative to 1 mol of potassium ions, and the adsorbent using γ-cyclodextrin (Comparative Example 2) clearly show that the adsorption performance of the adsorbent of Example 1 is significantly improved.

On the other hand, the organic ligand itself does not necessarily need to have a function capable of encapsulating the guest molecule. Any substance may be used as long as the metal-organic framework as a whole has a function capable of encapsulating the guest molecule. Examples of such an organic ligand include a compound having an imidazole backbone (such as imidazole, 2-methylimidazole, and 2-formylimidazole), and a compound having a monocyclic or polycyclic backbone (such as terephthalic acid and trimesic acid).

According to the present embodiment, it is possible to achieve a metal-organic framework excellent in guest molecule adsorption properties. According to the present embodiment, a metal-organic framework having a nitrogen gas adsorption amount of, for example, 220 cm³/g or more is achieved. Such a metal-organic framework is useful as an adsorbent for adsorbing nitrogen gas. In a preferred embodiment, a metal-organic framework having a nitrogen gas adsorption amount of 250 cm³/g or more can be achieved. In a more preferred embodiment, a metal-organic framework having a nitrogen gas adsorption amount of 280 cm³/g or more can be achieved. The nitrogen gas adsorption amount can be measured by the method described in Examples provided later.

Further, according to the present embodiment, a metal-organic framework having a CO₂ gas adsorption amount of, for example, 10 cm³/g or more is achieved. Such a metal-organic framework is useful as an adsorbent for adsorbing CO₂ gas. In a preferred embodiment, a metal-organic framework having a CO₂ gas adsorption amount of 20 cm³/g or more can be achieved. In a more preferred embodiment, a metal-organic framework having a CO₂ gas adsorption amount of 40 cm³/g or more can be achieved. The CO₂ gas adsorption amount can be measured by the method described in Examples provided later.

CO₂ gas adsorption occurs when the pore size of the metal-organic framework is approximately the same as or larger than the molecular diameter of the CO₂ gas. In particular, when a polar group such as a hydroxyl group, a carboxyl group, or an amino group is present in the organic ligand constituting the metal-organic framework, an interaction occurs between the pore wall surface of the metal-organic framework and carbon dioxide, thereby improving the CO₂ gas adsorption performance. For example, when cyclodextrin is used as the organic ligand, the primary alcohol of glucose constituting cyclodextrin interacts with CO₂ gas, and CO₂ gas is adsorbed as carboxylic acid. Therefore, the maximum CO₂ gas adsorption amount depends on the pore specific surface area of the metal-organic framework and/or the abundance of free polar groups that are not involved in the structural constitution, present in the organic ligand constituting the metal-organic framework.

The pore specific surface area of the metal-organic framework is, for example, 700 to 1400 m²/g, preferably 800 to 1300 m²/g, more preferably 900 to 1200 m²/g. The pore specific surface area can be measured by the method described in Examples described later.

The pore volume of the metal-organic framework is, for example, 0.300 to 0.600 cm³/g, preferably 0.350 to 0.500 cm³/g. The pore volume can be measured by the method described in Examples provided later.

The pore diameter of the metal-organic framework is, for example, 1.300 to 1.600 nm, preferably 1.400 to 1.500 nm. The pore diameter can be measured by the method described in Examples provided later.

### 2: Method of producing metal-organic framework

The metal-organic framework having the configuration as described above can be produced, for example, by using the specific production method described later.

That is, the production method according to the present embodiment includes a step (step S1) of preparing an aqueous solution containing a metal ion and an organic ligand capable of coordinating to the metal ion as a first solution, and directly adding a second solution containing an organic solvent to the first solution to produce a metal-organic framework in which the organic ligand is coordinated to the metal ion (step S2). Hereinafter, each step will be described in detail.

### (Step S1: Preparation of First Solution)

First, as a first solution, an aqueous solution containing a metal ion and an organic ligand is prepared. Specifically, a metal compound and an organic ligand are added to water and dissolved. Thereby, the first solution can be obtained. The first solution can be prepared at room temperature (1 to 30°C).

The metal compound used as the metal ion source is not particularly limited, and examples thereof include metal hydroxides, inorganic halide salts such as chloride salts, and inorganic acid salts such as nitrates and acetates.

When the metal ion is an alkali metal ion, it is preferable to use an alkali metal hydroxide as the metal compound.

When the metal ion is a Zn ion or an Fe ion, it is preferable to use an inorganic acid salt as the metal compound.

The pH of the first solution may be any pH at which the metal in the metal compound exists as an ion.

For example, when an alkali metal ion is used as the metal ion, the first solution is alkaline. In this case, the pH of the first solution is, for example, 8 or more, preferably 10 to 14, more preferably 13 to 14.

When a Zn ion is used as the metal ion, the suitable pH varies depending on the type of the zinc compound serving as the ion source, but when zinc acetate is used as the source, it is, for example, 6 to 13, preferably 7 to 12, more preferably 9 to 10.

Also when an Fe ion is used as the metal ion, a suitable pH varies depending on the type of the iron compound serving as the ion source, but when iron(III) nitrate is used, it is, for example, 1 to 6, preferably 1 to 4, more preferably 2 to 3.

In the first solution, the amount of the organic ligand relative to 1 mol of the metal ion is, for example, 0.05 to 50 mol, preferably 0.1 to 40 mol, more preferably 0.125 to 30 mol.

When the metal ion is an alkali metal ion, the amount of the organic ligand relative to 1 mol of the metal ion is preferably 0.05 to 8 mol, more preferably 0.125 to 8 mol, and still more preferably 0.125 to 0.8 mol.

When the metal ion is a potassium ion and the organic ligand is a cyclodextrin-based compound, as described above, it is preferably in the range of 0.05 to 0.8, and particularly in the case where the organic ligand is α-cyclodextrin, it is preferably in the range of 0.125 to 0.375, and in the case of γ-cyclodextrin, it is preferably in the range of 0.125 to 0.750.

When the metal ion is a Zn ion, the amount of the organic ligand relative to 1 mol of the metal ion is preferably 0.5 to 50 mol, more preferably 15 to 40 mol.

When the metal ion is an Fe ion, the amount of the organic ligand relative to 1 mol of the metal ion is preferably 0.05 to 5 mol, more preferably 0.1 to 1 mol.

Within the range as described above, it becomes possible to obtain a metal-organic framework excellent in the adsorption properties for guest molecules such as nitrogen gas.

The amount of water relative to 1 mol of the metal ion is, for example, 10 to 5000 mol, preferably 50 to 5000 mol.

When an alkali metal ion is used as the metal ion, the amount of water relative to 1 mol of the metal ion is preferably 50 to 200 mol, more preferably 80 to 200 mol, and still more preferably 100 to 150 mol.

When a Zn ion is used as the metal ion, the amount of water relative to 1 mol of the metal ion is preferably 500 to 5000 mol, and preferably 1000 to 3500 mol.

When an Fe ion is used as the metal ion, the amount of water relative to 1 mol of the metal ion is preferably 200 to 3000 mol, and preferably 500 to 2000 mol.

The molar ratio of the organic ligand to water (organic ligand:water) is, for example, 1:10 to 1:2000, preferably 1:50 to 1:2000, more preferably 1:50 to 1:1500, further preferably 1:80 to 1:1500, and most preferably 1:100 to 1:1300. When the organic ligand is a cyclodextrin-based compound, it is preferably in the range of 1:100 to 1:1300.

### (Step S2: Addition of Second Solution)

Subsequently, a second solution is directly added to the first solution. Here, "directly" adding means adding the second solution to the first solution while it is in a liquid state.

The second solution contains an organic solvent. The second solution is preferably a solution that changes the pH of the first solution.

More preferably, the second solution is a solution that lowers the pH of the first solution.

Examples of such an organic solvent include solvents that deprotonate the coordination bonding site in the organic ligand. Examples of the organic solvent include at least one solvent selected from the group consisting of alcohol-based solvents, ketone-based solvents, and aprotic solvents. By adding such an organic solvent, the organic ligand is deprotonated, and as a result, a coordination bond is formed between the metal ion and the organic ligand, and a metal-organic framework is formed.

In this case, the second solution is preferably added in such an amount that the pH of the first solution decreases by, for example, 0.01 to 0.20, preferably 0.05 to 0.15.

Examples of the alcohol-based solvent include methanol, ethanol, 1-propanol, and 1-butanol.

Examples of the ketone-based solvent include acetone.

Examples of the aprotic solvent include N,N-dimethylformamide.

A preferred organic solvent is an alcohol-based solvent, more preferably methanol.

Preferably, the second solution is added to the first solution while applying shear to the first solution. More preferably, the second solution is added to the first solution while stirring the first solution. The second solution can be added to the first solution at room temperature.

It is preferable to add the second solution to the first solution over, for example, 1 to 20 minutes, preferably 10 to 15 minutes.

When all of the second solution is added at once, clouding may occur, and the desired metal-organic structure may not be obtained. On the other hand, by adding over 1 to 20 minutes, nuclei can be formed and the crystal structure can be grown, so that the desired metal-organic structure can be easily obtained.

Examples of the method of adding the second solution include a method of adding at a constant speed using a dropping funnel, a method of adding a certain amount every predetermined time, a method of adding while changing the addition amount in a gradient manner, etc.

After the addition of the second solution, preferably, the mixed solution of the first solution and the second solution is further stirred. The mixed solution is stirred at room temperature for, for example, 5 to 50 hours, preferably 10 to 30 hours after the addition of the second solution. When stirring is not performed, a two-layer separation state having a cloudy lower layer and a transparent upper layer may occur, and it may not be possible to eliminate the two-layer separation state.

The amount of the organic solvent added is, for example, 3 to 1000 mol, preferably 5 to 500 mol, relative to 1 mol of the metal ion.

When the metal ion is an alkali metal ion and the organic ligand is γ-cyclodextrin, the amount of the organic solvent added is preferably 3 to 100 mol, preferably 5 to 50 mol, and more preferably 10 to 30 mol relative to 1 mol of the metal ion.

When the metal ion is potassium ion and the organic ligand is α-cyclodextrin, the organic solvent is suitably added in an amount in the range of preferably 10 to 400 mol, preferably 50 to 300 mol, more preferably 100 to 200 mol relative to 1 mol of potassium ions; in the case of γ-cyclodextrin, the organic solvent is suitably added in an amount in the range of preferably 3 to 100 mol, preferably 5 to 50 mol, and more preferably 10 to 30 mol relative to 1 mol of potassium ions.

When the metal ion is a Zn ion, the amount of the organic solvent added is preferably 50 to 500 mol, more preferably 75 to 200 mol, relative to 1 mol of the metal ion.

When the metal ion is an Fe ion, the amount of the organic solvent added is preferably 100 to 800 mol, more preferably 250 to 500 mol, relative to 1 mol of the metal ion.

By the method described above, a metal-organic framework can be produced. The metal-organic framework is produced as a solid product. Therefore, the metal-organic framework can be isolated by, for example, filtering the mixed solution. After filtration, the isolated metal-organic framework may be further washed with an organic solvent such as methanol. Also, the isolated metal-organic framework may be dried.

According to the method according to the present embodiment described above, a metal-organic framework excellent in guest molecule adsorption properties can be synthesized in a short time.

### (Adsorbent)

The adsorbent of the present invention contains a metal-organic framework having a cyclic structure or a cage-like structure formed by coordinating at least one organic ligand to at least one metal ion, and the organic ligand is a cyclic organic compound; therefore, it is possible to provide an adsorbent having excellent adsorption performance.

The adsorbent of the present invention can efficiently adsorb monocyclic compounds, particularly cyclopentane derivatives.

Further, the adsorbent of the present invention can exhibit excellent adsorption performance even when blended in a resin.

The metal-organic framework constituting the adsorbent of the present invention has a cyclic or cage-like porous crystal structure in which at least one organic ligand is coordinated to at least one metal ion, and since the space structure inside the crystal is uniform, guest molecules can be efficiently encapsulated, and excellent performance as an adsorbent can be exhibited.

Further, in the metal-organic framework used in the present invention, it is also important that the metal-organic framework uses a cyclic organic compound as an organic ligand. That is, in the metal-organic framework used in the present invention, unlike the metal-organic framework used in Patent Literature 1, a cyclic organic compound such as an aromatic organic compound or a heterocyclic organic compound is not used as an organic ligand; rather, it means an organic compound having a cyclic structure such as a cyclodextrin-based compound. Cyclodextrin-based compounds themselves have a structure capable of encapsulating guest molecules, so that they can further improve adsorption performance; as naturally occurring compounds, they are also excellent in environmental friendliness.

The adsorbent of the present invention only needs to contain at least the metal-organic framework having a cyclic structure or a cage-like structure described above, and can be used alone as an adsorbent, but that is not intended to exclude other components. For example, in addition to the above metal-organic framework, other known porous materials such as metal-organic frameworks and zeolite may be contained as necessary.

The form of the adsorbent can be powder, particulate, granular, etc., as mentioned above in reference to the metal-organic frameworks; alternatively, as will be described later, it may also be molded into shapes such as pellets, fibers, membranes, or films when incorporated into a resin composition.

The adsorbent of the present invention can be used by known methods; it can be added directly to the target material for use, formed into particles or pellets and filled into a column or similar apparatus, or shaped into a membrane or film to be used as a filter.

The adsorbent of the present invention can adsorb at least monocyclic compounds. Examples of such monocyclic compounds include cyclopentane derivatives and cyclohexane derivatives.

Examples of the cyclopentane derivative include methylcyclopentane, cyclopentene, cyclopentadiene, and cyclopentanol, and examples of the cyclohexane derivative include methylcyclohexane, cyclohexanedicarboxylic acid, and cyclohexanedimethanol.

Further, in the case of a metal-organic framework composed of potassium ions as metal ions and α-cyclodextrin or γ-cyclodextrin as organic ligands (hereinafter sometimes referred to as "α-CD-MOF" or "γ-CD-MOF", respectively), in addition to the above-mentioned monocyclic compounds, adsorption performance can be exhibited for butanal, ethyl acetate, hexane and its derivatives, formic acid, and the like. Further, α-CD-MOF can exhibit adsorption performance for octane and its derivatives, octene and its derivatives, in addition to the above, and γ-CD-MOF can exhibit adsorption performance for heptene derivatives in addition to the above.

### (Resin Composition)

The resin composition of the present invention is a resin containing the above adsorbent.

Conventional resins, which are well-known and not limited to specific types, can be employed to include the adsorbent, and examples thereof include, but are not limited to, low-density, medium-density, and high-density polyethylene, linear low-density polyethylene, linear ultra-low-density polyethylene, isotactic polypropylene, syndiotactic polypropylene, propylene-ethylene copolymer, polybutene-1, ethylene-butene-1 copolymer, propylene-butene-1 copolymer, ethylene-propylene-butene-1 copolymer, and other olefinic resins; polyester resins such as polyethylene terephthalate, polybutylene terephthalate, and polyethylene naphthalate; polyamide resins such as nylon 6, nylon 6,6, and nylon 6,10; thermoplastic resins such as polycarbonate; and thermosetting resins such as phenolic resins, epoxy resins, urethane resins, melamine resins, urea resins, alkyd resins, unsaturated polyester resins, and silicone resins, as well as UV-curable acrylic resins.

In the resin composition of the present invention, in order for the adsorbent in the resin composition to be able to efficiently exhibit its adsorption performance, the resin serving as the matrix is preferably a resin excellent in gas permeability, and among the above resins, particularly low-density polyethylene can be suitably used.

The content of the adsorbent in the resin composition can vary depending on the type of resin, application, etc., making it difficult to define a specific amount; however, it is preferred that the adsorbent be included in an amount ranging from 0.01 to 50 parts by mass per 100 parts by mass of resin, with a particular preference for 5 to 20 parts by mass. If the amount of adsorbent is below this range, there is a risk that the desired adsorptive properties may not be achieved; conversely, if the adsorbent content exceeds this range, the moldability of the resin composition into the desired form may be adversely affected.

The resin composition may contain a dispersant, an antioxidant, a filler, and a conventionally known resin additive in addition to the adsorbent.

The preparation of the resin composition can be carried out by conventionally known methods, though it is not limited to these; examples include dry blending the adsorbent with the resin, adding the adsorbent to the resin in its molten state and then blending, or dispersing the adsorbent in an organic solvent to create a dispersion, which is then added to the resin in its molten state for melt mixing.

The resin composition of the present invention can be formed into various forms depending on its applications, such as pellets, films, and sheets, as well as cups, trays, bottles, tubes, etc.

### Examples

Hereinafter, in order to describe the present invention in more detail, Examples conducted by the present inventors will be described.

### (Measurement Method)

### [Thermogravimetric Analysis Evaluation]

The combustion behavior and potassium residual amount of the metal-organic framework were measured by thermogravimetric analysis using a TG/DTA 7220 manufactured by Hitachi, Ltd. at a heating rate of 3°C/min and a measurement temperature of 30°C to 500°C.

### [Molecular Structure Evaluation]

The molecular structure of the combustion residue of the metal-organic framework generated by the thermogravimetric analysis evaluation was analyzed by Raman spectroscopy using a DXR manufactured by Thermo Scientific, optical system DXR Raman Microscope, laser: 532 nm, laser output: 5.0 mW, exposure time: 6.00 seconds, number of exposures: 10.

### [Composition Molar Ratio]

As a result of the thermogravimetric analysis evaluation, the combustion residue was considered to be potassium bicarbonate, and therefore the amount of potassium in the combustion residue was calculated. From the calculation result of the potassium amount, the molar ratio of γ-cyclodextrin to potassium in the metal-organic framework was identified by calculating γ-cyclodextrin in the metal-organic framework.

### [Specific Surface Area, Pore Volume, Pore Diameter, and Adsorption Amount]

The specific surface area, pore volume, pore diameter, and maximum nitrogen gas adsorption amount of the metal-organic framework were measured using a BELSORP MAX II manufactured by MicrotracBEL Corp.; the adsorption isotherm of nitrogen gas at liquid nitrogen temperature was obtained using the multipoint method, and the values were calculated through MP analysis.

Further, the maximum CO₂ gas adsorption amount was measured by measuring the CO₂ gas adsorption isotherm at 298 K and a measurement pressure in the range of 0 to 100 kPa by the multipoint method using a BELSORP MAX II manufactured by MicrotracBEL Corp.

### [Crystal Structure]

The crystal structure of the obtained metal-organic framework was evaluated by powder X-ray diffraction (PXRD) using SmartLab manufactured by Rigaku Corporation.

### [Adsorption Test on Adsorbent in Powder Form]

An adsorption sample was prepared by filling 0.2 g of each adsorbent into a collection bottle that trapped various odor components. Using GC/MS (GC-2010, manufactured by SHIMADZU Corporation), comparison and evaluation were carried out in terms of reduction rate using the TIC (total ion chromatogram) total area values of hydrocarbon compounds, monocyclic compounds, and alcohol compounds detected between retention times of 12.5 minutes and 22.5 minutes.

Analysis conditions: Column: DB-624 (60 m×0.25 mm×1.4 µm), column temperature: initial temperature of 40°C, held for 10 minutes, and increased at 10°C/min. After reaching the final temperature of 250°C, hold for 5 minutes, column flow rate: 1.88 ml/min, carrier gas: helium, ionization method: EI, ion source temperature: 200°C, detection: MS scan (m/z 26.0-550)
The adsorbed gas was identified from the obtained spectrum.

### [Adsorption Test of Adsorbent-Containing Resin Composition]

An adsorption sample was prepared by filling 2 g of the resin composition into a flask filled with 480 ppm of hexane, 500 ppm of octane, or 2 ppm of acetic acid. The concentration of each gas component 1 hour after filling the adsorption sample was measured with the following detector tubes.
Hexane concentration: detector tube (Gastec Corporation, No. 105)
Octane concentration: detector tube (Gastec Corporation, No. 105)
Acetic acid concentration: detector tube (Gastec Corporation, No. 81L)

### (Example 1)

After adding pure water to a 150 ml container (mayonnaise bottle), potassium hydroxide was added to the container and dissolved at room temperature. Further, γ-cyclodextrin was added and dissolved at room temperature. The pH of the resulting solution was 13.50. Next, methanol was added dropwise to this solution over about 15 minutes while stirring the solution using a stirrer. After the addition, the solution was stirred for 24 hours to obtain a suspension containing a solid product. The pH of the solution after the addition of methanol was 13.40. The solid product was separated from the obtained suspension by filtration, and the isolated solid product was washed with methanol. After washing, the solid product was dried at 50°C overnight to obtain metal-organic framework A according to Example 1.

The molar ratio of each component was potassium ion:y-cyclodextrin:water:methanol=1:0.125:137.5:20.

From the Raman spectroscopy of the combustion residue obtained from the TG measurement of the obtained metal-organic framework A, a peak of CC could be observed at 1050 to 1070 cm⁻¹, and the combustion residue was identified as potassium carbonate.

When the composition molar ratio of the obtained metal-organic framework A was calculated from the above measurement results, it was γ-cyclodextrin:potassium ion=1:6.70.

The pore specific surface area was 1327.1 m²/g. The pore volume was 0.483 cm³/g. The pore diameter was 1.457 nm. The maximum nitrogen gas adsorption amount was 312 cm³/g, and the maximum CO₂ gas adsorption amount was 57 cm³/g.

An adsorption sample was prepared by filling 0.2 g of metal-organic framework A into a collection bottle that trapped various odor components. Using GC/MS (GC-2010, manufactured by SHIMADZU Corporation), comparison and evaluation were carried out in terms of reduction rate using the TIC (total ion chromatogram) total area values of hydrocarbon compounds, monocyclic compounds, and alcohol compounds detected between retention times of 10.0 minutes and 30.0 minutes. The TIC total area value reduction rate was 86.3%.

GC/MS measurement results showed that the metal-organic framework A particularly adsorbed hydrocarbons, butanal, ethyl acetate, hexane, hexane derivatives, heptane, heptane derivatives, cyclopentane derivatives, formic acid, octane, octane derivatives, octene, and octene derivatives.

### (Example 2)

A metal-organic framework B was obtained in the same manner as in Example 1, except that the molar ratio of each component used was potassium ion:y-cyclodextrin:water:methanol=1:0.25:137.5:20.

When the composition molar ratio of the obtained metal-organic framework B was calculated in the same manner as in Example 1, it was γ-cyclodextrin:potassium ion=1:7.03.

The pore specific surface area was 1329.0 m²/g. The pore volume was 0.483 cm³/g. The pore diameter was 1.454 nm. The maximum nitrogen gas adsorption amount was 312 cm³/g, and the maximum CO₂ gas adsorption amount was 63 cm³/g.

The TIC total area value reduction rate was 90.3%. The TIC total area value reduction rate of Example 2 was also calculated in the same manner as in Example 1.

GC/MS measurement results showed that the metal-organic framework B particularly adsorbed hydrocarbons, butanal, ethyl acetate, hexane, hexane derivatives, heptane, heptane derivatives, cyclopentane derivatives, formic acid, octane, octane derivatives, octene, and octene derivatives.

### (Example 3)

A metal-organic framework C was obtained in the same manner as in Example 1, except that the molar ratio of each component used was potassium iοn:γ-cyclodextrin:water:methanol=1:0.375:137.5:20.

When the composition molar ratio of the obtained metal-organic framework C was calculated in the same manner as in Example 1, it was γ-cyclodextrin:potassium ion=1:7.09.

The pore specific surface area was 1243.8 m²/g. The pore volume was 0.452 cm³/g. The pore diameter was 1.453 nm. The maximum nitrogen gas adsorption amount was 291 cm³/g, and the maximum CO₂ gas adsorption amount was 54 cm³/g.

The TIC total area value reduction rate was 94.6%. The TIC total area value reduction rate of Example 3 was also calculated in the same manner as in Example 1.

GC/MS measurement results showed that the metal-organic framework C particularly adsorbed hydrocarbons, butanal, ethyl acetate, hexane, hexane derivatives, heptane, heptane derivatives, cyclopentane derivatives, formic acid, octane, octane derivatives, octene, and octene derivatives.

### (Example 4)

A metal-organic framework D was obtained in the same manner as in Example 1, except that the molar ratio of each component used was potassium ion:y-cyclodextrin:water:methanol=1:0.5 :137.5 :20.

When the composition molar ratio of the obtained metal-organic framework D was calculated in the same manner as in Example 1, it was γ-cyclodextrin:potassium ion=1:7.29.

The pore specific surface area was 1263.3 m²/g. The pore volume was 0.457 cm³/g. The pore diameter was 1.448 nm. The maximum nitrogen gas adsorption amount was 297 cm³/g, and the maximum CO₂ gas adsorption amount was 12 cm³/g.

The TIC total area value reduction rate was 92.5%. The TIC total area value reduction rate of Example 4 was also calculated in the same manner as in Example 1.

GC/MS measurement results showed that the metal-organic framework D particularly adsorbed hydrocarbons, butanal, ethyl acetate, hexane, hexane derivatives, heptane, heptane derivatives, cyclopentane derivatives, formic acid, octane, octane derivatives, octene, and octene derivatives.

### (Comparative Example 1)

A metal-organic framework H was synthesized according to the method described in Non-Patent Literature 1. Specifically, after adding pure water to a 30 cc container (screw tube bottle), potassium hydroxide (metal component) was added to the container and dissolved at room temperature. Further, γ-cyclodextrin (ligand component) was added and dissolved at room temperature. The molar ratio of each component at this time was potassium ion:γ-cyclodextrin:water=1:0.125:137.5.

Next, the container was placed in a 450 ml mayonnaise bottle containing methanol and sealed, and allowed to stand for 7 days; a suspension containing the solid product was obtained through the synthesis reaction at the liquid-liquid interface with methanol vapor.

Further, the solid product was separated from the obtained suspension by filtration, washed with the solid product, and dried at 50°C overnight. Thereby, the metal-organic framework H according to Comparative Example 1 was obtained.

When the composition molar ratio of the obtained metal-organic framework H was calculated in the same manner as in Example 1, it was γ-cyclodextrin:potassium ion=1:7.43.

The pore specific surface area was 853.3 m²/g. The pore volume was 0.31 cm³/g. The pore diameter was 1.451 nm. The maximum adsorption amount was 200 cm³/g.

### (Results)

Table 1 shows the results. As shown in Table 1, the composition molar ratio (organic ligand:metal) of the metal-organic framework according to Comparative Example 1 was 1:7.43, and the maximum nitrogen gas adsorption amount was 200 cm³/g. In contrast, in Examples 1 to 4, it was confirmed that 7.40 mol or less of alkali metal (potassium) was bonded to 1 mol of the organic ligand, and the maximum nitrogen gas adsorption amount was 220 cm³/g or more, which was superior to Comparative Example 1 in nitrogen gas adsorption properties. Moreover, in Examples 1 to 4, it was confirmed that the CO₂ gas adsorption amount was 10 cm³/g or more, and that the metal-organic frameworks had excellent CO₂ gas adsorption capacity.

**Table 1**

| | Amount of Metal Added (Potassium: K) | Organic Ligand (γ-CD) (Amount Added) | Composition Molar Ratio (Organic Ligand Componml:Mctal) | Max Nilogen Gas Adsorption Amount (cm³/g) | Max CO₂ Gas Adsorption Amount (cm³/g) |
|---|---|---|---|---|---|
| Example 1 | 8 mmol | 1 mmol | 1:6.70 | 312 | 57 |
| Example 2 | ↑ | 2 mmol | 1:7.03 | 312 | 63 |
| Example 3 | ↑ | 3 mmol | 1:7.09 | 291 | 54 |
| Example 4 | ↑ | 4 mmol | 1:7.29 | 297 | 12 |
| Comparative Example 1 | ↑ | 1 mmol | 1:7.43 | 200 | - |

### (Example 5)

A metal-organic framework E was obtained in the same manner as in Example 1, except that the molar ratio of each component used was potassium ion:γ-cyclodextrin:water:methanol=1:0.625:137.5:20.

The pore specific surface area was 1091.0 m²/g. The pore volume was 0.395 cm³/g. The pore diameter was 1.450 nm. The maximum adsorption amount was 258 cm³/g.

The TIC total area value reduction rate was 87.6%. The TIC total area value reduction rate of Example 5 was also calculated in the same manner as in Example 1.

GC/MS measurement results showed that the metal-organic framework E particularly adsorbed hydrocarbons, butanal, ethyl acetate, hexane, hexane derivatives, heptane, heptane derivatives, cyclopentane derivatives, formic acid, octane, octane derivatives, octene, and octene derivatives.

### (Example 6)

A metal-organic framework F was obtained in the same manner as in Example 1, except that the molar ratio of each component used was potassium ion:y-cyclodextrin:water:methanol=1:0.75:137.5:20.

The pore specific surface area was 651.4 m²/g. The pore volume was 0.236 cm³/g. The pore diameter was 1.449 nm. The maximum adsorption amount was 157 cm³/g.

The TIC total area value reduction rate was 87.1%. The TIC total area value reduction rate of Example 6 was also calculated in the same manner as in Example 1.

GC/MS measurement results showed that the metal-organic framework F particularly adsorbed hydrocarbons, butanal, ethyl acetate, hexane, hexane derivatives, heptane, heptane derivatives, cyclopentane derivatives, formic acid, octane, octane derivatives, octene, and octene derivatives.

### (Example 7)

A metal-organic framework G was obtained in the same manner as in Example 1, except that the metal component was sodium hydroxide.

The pore specific surface area was 945.5 m²/g. The pore volume was 0.345 cm³/g. The pore diameter was 1.461 nm. The maximum adsorption amount was 226 cm³/g.

### (Consideration of Examples 1 to 7 and Comparative Example 1)

Examples 1 to 7 had pore specific surface area, pore volume, pore diameter, and maximum adsorption amount comparable to those of Comparative Example 1. In Comparative Example 1, it took at least 7 days to produce the crystalline substance needed to obtain the metal-organic framework, whereas in Examples 1 to 7, the metal-organic framework could be obtained in one day.

### (Example 8)

The same procedure as in Example 1 was repeated, except that the organic ligand was α-cyclodextrin, and the molar ratio of each component used was potassium ion:α-cyclodextrin: water: methanol= 1: 0. 125:137.5: 184; as a result, a metal-organic framework I was obtained.

The obtained metal-organic framework I was confirmed to have a crystal structure by PXRD.

When the composition molar ratio of the obtained metal-organic framework I was calculated in the same manner as in Example 1, it was α-cyclodextrin:potassium ion=1:3.78.

The TIC total area value reduction rate was 97.9%. The TIC total area value reduction rate of Example 8 was also calculated in the same manner as in Example 1.

GC/MS measurement results showed that the metal-organic framework I particularly adsorbed hydrocarbons, butanal, ethyl acetate, hexane, hexane derivatives, heptane, heptane derivatives, cyclopentane derivatives, formic acid, octane, octane derivatives, octene, and octene derivatives.

### (Example 9)

The same procedure as in Example 1 was repeated, except that the organic ligand was β-cyclodextrin, and the molar ratio of each component used was potassium ion:β-cyclodextrin: water: methanol= 1: 0. 125:137.5:104; as a result, a metal-organic framework J was obtained.

The obtained metal-organic framework J was confirmed to have a crystal structure by PXRD.

### (Comparative Example 2)

Using GC/MS (GC-2010, manufactured by SHIMADZU Corporation) in the same manner as in Example 1 except that γ-cyclodextrin was filled inside the collection bottle, comparison and evaluation were carried out in terms of reduction rate using the TIC total area value. The TIC total area value reduction rate was 5.6%. The TIC total area value reduction rate of Comparative Example 2 was calculated based on the TIC total area values in a configuration identical to Example 1, except that no adsorbent was dispersed within the collection bottle that trapped various odor components.

### (Comparative Example 3)

Using GC/MS (GC-2010, manufactured by SHIMADZU Corporation) in the same manner as in Example 1 except that α-cyclodextrin was filled inside the collection bottle, comparison and evaluation were carried out in terms of reduction rate using the TIC total area value. The TIC total area value reduction rate was 18.4%. The TIC total area value reduction rate of Comparative Example 3 was calculated based on the TIC total area values in a configuration identical to Example 1, except that no adsorbent was dispersed within the collection bottle that trapped various odor components.

### (Example 10)

After adding pure water to a 150 ml container (mayonnaise bottle), 2-methylimidazole was added to the container and dissolved at room temperature (pH of the solution at this time was 9.64), and an aqueous solution of zinc acetate dihydrate (zinc acetate dihydrate:pure water=0.27 g:5 g) was further added. The pH of the resulting solution was 7.48. Next, methanol was added dropwise to this solution while stirring the solution using a stirrer. After the addition, the solution was stirred for 1 hour to obtain a suspension containing a solid product. The pH of the solution after the addition of methanol was 7.38. The solid product was separated from the obtained suspension by filtration, and the isolated solid product was washed with methanol. After washing, the solid product was dried at 50°C overnight to obtain metal-organic framework K according to Example 1.

The molar ratio of each component was zinc ion:2-methylimidazole:water:methanol=1:2:2220:125.

The pore specific surface area was 69.9 m²/g. The pore volume was 0.015 cm³/g. The pore diameter was 0.879 nm. The maximum adsorption amount was 65 cm³/g.

### (Example 11)

A metal-organic framework L was obtained in the same manner as in Example 10, except that the molar ratio of each component used was zinc ion:2-methylimidazole:water:methanol=1:18:2220:125.

The pore specific surface area was 1610.5 m²/g. The pore volume was 0.563 cm³/g. The pore diameter was 1.398 nm. The maximum adsorption amount was 351 cm³/g.

### (Example 12)

A metal-organic framework M was obtained in the same manner as in Example 10, except that the molar ratio of each component used was zinc ion:2-methylimidazole:water:methanol=1:30:2220:125.

The pore specific surface area was 1674.0 m²/g. The pore volume was 0.583 cm³/g. The pore diameter was 1.394 nm. The maximum adsorption amount was 367 cm³/g.

### (Example 13)

After adding pure water to a 150 ml container (mayonnaise bottle), trimesic acid and iron(III) nitrate nonahydrate were added to the container. The pH of the resulting solution was 2.11.

Next, methanol was added dropwise to this solution while stirring the solution using a stirrer. After the addition, the solution was stirred for 48 hours to obtain a suspension containing a solid product. The pH of the solution after the addition of methanol was 2.03. The solid product was separated from the obtained suspension by filtration, and the isolated solid product was washed with methanol. After washing, the solid product was dried at 50°C overnight to obtain metal-organic framework N according to Example 1.

The molar ratio of each component was iron ion:trimesic acid: water: methanol= 1: 0. 25:1247:3 52.

The pore specific surface area was 1068.6 m²/g. The pore volume was 0.42 cm³/g. The pore diameter was 1.579 nm. The maximum adsorption amount was 277 cm³/g.

### (Example 14)

A metal-organic framework L was obtained in the same manner as in Example 13, except that the molar ratio of each component used was iron ion:trimesic acid:water:methanol=1:0.5:1247:352.

The pore specific surface area was 806.9 m²/g. The pore volume was 0.298 cm³/g. The pore diameter was 1.476 nm. The maximum adsorption amount was 284 cm³/g.

### (Example 15)

A metal-organic framework O was obtained in the same manner as in Example 13, except that the molar ratio of each component used was iron ion:trimesic acid: water: methanol= 1:0.67:1247:352.

The pore specific surface area was 145.7 m²/g. The pore volume was 0.051 cm³/g. The pore diameter was 0.700 nm. The maximum adsorption amount was 39 cm³/g.

### (Consideration of Examples 8 to 15)

As shown in Examples 8 to 15, even when a metal ion other than an alkali metal ion was used as the metal ion and a substance different from γ-cyclodextrin was used as the organic ligand, it was possible to obtain an organometallic framework in a short time, as in Examples 1 to 7.

### (Example 16)

The following raw materials were mixed to obtain an adsorbent-containing resin composition.
LDPE (Novatec LC600A): 90 parts
Metal-organic framework A: 10 parts

Then, 2 g of the resin composition prepared above was formed into a film having an average film thickness of 0.2 mm using a pressing machine.

After drying the prepared film at 140°C for 3 hours, 2 g of the film was filled into a flask filled with 480 ppm of hexane, 500 ppm of octane, or 2 ppm of acetic acid to prepare an adsorption sample. The gas component concentration after 1 hour of filling the adsorption sample was measured with a detector tube. An adsorption test was conducted, and as a result, the film made of the gas adsorbent-containing resin composition adsorbed 96 ppm of hexane, 150 ppm of octane, and 2 ppm of acetic acid.

### (Comparative Example 4)

The following materials were mixed to obtain a resin composition.
LDPE (Novatec LC600A): 100 parts

Then, 2 g of the resin composition prepared above was formed into a film having an average film thickness of 0.2 mm using a pressing machine.

The adsorption measurement test was carried out in the same manner as in Example 3, and the film adsorbed 16 ppm of hexane, 100 ppm of octane, and 0.2 ppm of acetic acid.

## Claims

1. A metal-organic framework comprising a metal and an organic ligand coordinated to the metal, wherein
the metal-organic framework has a cyclic structure or a cage-like structure, and
the metal is bonded in an amount of 7.40 mol or less relative to 1 mol of the organic ligand.

2. The metal-organic framework according to claim 1, wherein a nitrogen gas adsorption amount is 220 cm³/g or more.

3. The metal-organic framework according to claim 1, wherein a CO₂ gas adsorption amount is 10 cm³/g or more.

4. The organometallic framework according to claim 1, wherein the metal is an alkali metal.

5. The organometallic framework according to claim 4, wherein the alkali metal is potassium.

6. The organometallic framework according to claim 1, wherein the organic ligand is cyclodextrin.

7. The organometallic framework according to claim 6, wherein the cyclodextrin is γ-cyclodextrin.

8. An adsorbent comprising the organometallic framework according to any one of claims 1 to 7.

9. An adsorbent capable of adsorbing at least a monocyclic compound, wherein the adsorbent contains a metal-organic framework having a cyclic structure or a cage-like structure formed by coordinating at least one organic ligand to at least one metal ion, and the organic ligand is a cyclic organic compound.

10. The adsorbent according to claim 9, wherein the metal ion is an alkali metal ion.

11. The adsorbent according to claim 10, wherein the alkali metal is potassium.

12. The adsorbent according to claim 9, wherein the cyclic organic compound is a cyclodextrin-based compound.

13. The adsorbent according to claim 12, wherein the cyclodextrin-based compound is α-cyclodextrin or γ-cyclodextrin.

14. The adsorbent according to claim 9, wherein the organic ligand of the metal-organic framework is cyclodextrin, and the metal ion is a potassium ion.

15. The adsorbent according to claim 9, wherein the metal-organic framework has a two-dimensional sheet-like layered structure.

16. The adsorbent according to claim 9, wherein the monocyclic compound is a cyclopentane derivative.

17. The adsorbent according to claim 9, wherein the metal ion is bonded in an amount of 7.40 mol or less relative to 1 mol of the organic ligand.

18. A resin composition comprising the adsorbent according to claim 9 in a resin.

19. A method of producing a metal-organic framework having a cyclic structure, the method comprising:
preparing an aqueous solution containing a metal ion and an organic ligand capable of coordinating to the metal ion as a first solution; and
directly adding a second solution containing an organic solvent to the first solution to produce a metal-organic framework in which the organic ligand is coordinated to the metal ion.

20. The production method according to claim 19, wherein the metal ion is bonded in an amount of 7.40 mol or less relative to 1 mol of the organic ligand.

21. The production method according to claim 19, wherein the metal ion is an alkali metal ion.

22. The production method according to claim 21, wherein the metal ion is a potassium ion.

23. The production method according to claim 19, wherein the organic ligand includes a cyclodextrin-based compound.

24. The production method according to claim 23, wherein the cyclodextrin-based compound includes γ-cyclodextrin.

25. The production method according to claim 19, wherein the metal ion is a zinc ion, and the organic ligand is a compound having an imidazole backbone.

26. The production method according to claim 25, wherein the compound having an imidazole backbone is 2-methylimidazole.

27. The production method according to claim 19, wherein the metal ion is an iron ion, and the organic ligand is a compound having a monocyclic or polycyclic backbone.

28. The production method according to claim 27, wherein the compound having a monocyclic or polycyclic backbone is trimesic acid.

29. The production method according to claim 19, wherein the organic ligand is used in an amount of 0.05 to 50 mol relative to 1 mol of the metal ion in the first solution.

30. The production method according to claim 19, wherein the second solution is a solution that changes the pH of the first solution.

31. The production method according to claim 30, wherein
the second solution is a solution that lowers the pH of the first solution, and
the second solution is added in an amount so that the pH of the first solution decreases by 0.01 to 0.20.

32. The production method according to claim 19, wherein the second solution contains at least one solvent selected from the group consisting of an alcohol-based solvent, a ketone-based solvent, and an aprotic solvent.

33. The production method according to claim 19, wherein in the step of producing the metal-organic framework, the second solution is added to the first solution while applying shear to the first solution.

34. The production method according to any one of claims 19 to 33, further comprising isolating the metal-organic framework after the step of producing the metal-organic framework.

35. The production method according to claim 34, wherein the isolating step includes filtering a mixed solution of the first solution and the second solution.

36. The production method according to claim 35, wherein the isolating step includes washing or drying the metal-organic framework after the filtering step.
